# EUROPEAN PATENT SPECIFICATION

(11) **EP 3 009 520 B1**
(45) Date of publication and mention of the grant of the patent: **12.12.2018**
(21) Application number: 14003525.4
(22) Date of filing: 14.10.2014
(51) Int. Cl.: C12Q 1/6806

(54) **Site-specific immobilization of DNA origami structures on solid substrates**
Stellenspezifische Immobilisierung von DNA-Origamistrukturen auf festen Substraten
Immobilisation spécifique par site de structures origami d'ADN sur des substrats solides

(43) Date of publication of application: 20.04.2016
(73) Proprietor: Karlsruher Institut für Technologie, 76131 Karlsruhe (DE)
(72) Inventor: Niemeyer, Christof, 28213 Bremen (DE); Meyer, Rebecca, 44141 Dortmund (DE); Angelin, Alessandro, 76131 Karlsruhe (DE); Foulkes, Nicholas S., 76344 Eggenstein (DE); Kassel, Olivier, F67270 Schwindratzheim (FR); Weigel, Simone, 76133 Karlsruhe (DE); Bauer, Jens, 76889 Vorderweidenthal (DE); Scharnweber, Tim, 76694 Forst (DE)
(74) Representative: Müller-Boré & Partner Patentanwälte PartG mbB

(56) References cited:
- AKISHIBA T ET AL: "DNA origami assembly on patterned silicon by AFM based lithography", MICRO ELECTRO MECHANICAL SYSTEMS (MEMS), 2013 IEEE 26TH INTERNATIONAL CONFERENCE ON, IEEE, 20 January 2013 (2013-01-20), pages 307-310, XP032339234, DOI: 10.1109/MEMSYS.2013.6474239 ISBN: 978-1-4673-5654-1
- ANTHONY C. PEARSON ET AL: "Chemical Alignment of DNA Origami to Block Copolymer Patterned Arrays of 5 nm Gold Nanoparticles", NANO LETTERS, vol. 11, no. 5, 11 May 2011 (2011-05-11), pages 1981-1987, XP055180180, ISSN: 1530-6984, DOI: 10.1021/nl200306w
- MAX B. SCHEIBLE ET AL: "Single Molecule Characterization of DNA Binding and Strand Displacement Reactions on Lithographic DNA Origami Microarrays", NANO LETTERS, vol. 14, no. 3, 12 March 2014 (2014-03-12) , pages 1627-1633, XP055180183, ISSN: 1530-6984, DOI: 10.1021/nl500092j
- ALBERT M. HUNG ET AL: "Large-area spatially ordered arrays of gold nanoparticles directed by lithographically confined DNA origami", NATURE NANOTECHNOLOGY, vol. 5, no. 2, 20 December 2009 (2009-12-20), pages 121-126, XP055180185, ISSN: 1748-3387, DOI: 10.1038/nnano.2009.450
- BARBARA SACCÀ ET AL: "Orthogonal Protein Decoration of DNA Origami", ANGEWANDTE CHEMIE INTERNATIONAL EDITION, vol. 49, no. 49, 3 December 2010 (2010-12-03), pages 9378-9383, XP055180190, ISSN: 1433-7851, DOI: 10.1002/anie.201005931
- CHHABRA R ET AL: "Spatially addressable multiprotein nanoarrays templated by aptamer-tagged DNA nanoarchitectures", JOURNAL OF THE AMERICAN CHEMICAL SOCIETY, AMERICAN CHEMICAL SOCIETY, US, vol. 129, no. 34, 29 August 2007 (2007-08-29), pages 10304-10305, XP002561874, ISSN: 0002-7863, DOI: 10.1021/JA072410U [retrieved on 2007-08-04]
- ALESSANDRO ANGELIN ET AL: "Multiscale Origami Structures as Interface for Cells", ANGEWANDTE CHEMIE INTERNATIONAL EDITION, vol. 54, no. 52, 7 December 2015 (2015-12-07), pages 15813-15817, XP055388975, ISSN: 1433-7851, DOI: 10.1002/anie.201509772

## Description

The present invention relates to methods for the site-specific immobilization of DNA origami structures on solid substrates.

The present invention is based on "Structural DNA nanotechnology" and "Scaffolded DNA Origami" techniques. The former approach, initiated in the early 1980s by Nadrian Seeman, takes advantage of the molecular recognition properties of synthetic DNA oligonucleotides to create finite size objects or infinite periodic lattices through complementary Watson-Crick base pairing, sticky-end cohesion and stacking interactions. Scaffolded DNA Origami, introduced in 2006 by Paul Rothemund, simplifies the access to finite DNA nanoobjects. In this technique, single-stranded circular DNA molecules of typically 7.25 kb size are folded by the aid of staple strands into an array of helices through an arrangement of periodic crossovers. Extension of this concept to assemble 3D DNA structures has led to the establishment of a robust and reliable method to fabricate DNA nanostructures with dimensions in the 20 to 100 nm range. Origami structures can be used as "molecular pegboards" with an addressable surface area of a few thousand nm² for arranging arbitrary objects-of-interest (OOI) with a "single pixel" resolution of about 6 nanometers. Typical OOls are metal or semiconductor nanoparticles for potential applications in microelectronics and (non-linear) optics, or proteins, peptides and other ligands for potential applications in biosensing, cell culture, or other areas of biological and biomedical research. In this context, DNA origami structures could advantageously allow for control of the absolute number, stoichiometry and precise nanoscale orientation of OOls, e.g. receptors and additional cell membrane proteins for presentation to living cells.

Many of the aforementioned potential applications require or would at least benefit from methods to immobilize DNA origami structures on solid supports, in particular in a site-specific manner. So far, nanoparticle-decorated origami structures have been deposited on nanopatterned surfaces prepared by electron-beam lithography and etching processes to create DNA origami-shaped binding sites on non-transparent substrates. While these approaches proved feasible for generating large-area spatially ordered arrays of DNA origami, the production of substrates is very cost and time consuming. Even more important, site-selectivity could not yet be realized because origami immobilization is governed by mere electrostatic interactions. Further, conventional top-down patterning techniques cannot be used for this purpose, because they either do not have sufficient lateral resolution or they do not allow for patterning of multiple different OOls such as different proteins. For instance, classical photolithography (resolution ∼ 1µm), X-ray- and electron-beam lithography (resolution ∼ 20 nm), or novel variants of unconventional high resolution lithography, such as block co-polymer micelle nanolithography which allow for production of < 10 nm feature sizes, can only be used for serial patterning of a single molecular component. The same holds true for microcontact printing and nanoimprinting (resolution ∼ 1µm and > 20 nm, respectively). Although direct writing of proteins with dip-pen nanolithography (DPN) has been described as a potential means to prepare protein feature sizes of > 50 nm, this technique has not yet proven its suitability to generate patterns of multiple different proteins with the required lateral resolution. Notably, direct writing of many different cell specific peptide and protein ligands by DPN is generally not feasible because different polypeptides necessitate individual buffer composition regarding pH, ion strength, detergents, and co-solvents required for the DPN process.

In this context, Akishiba *et al.* (Akishiba, T. et al.; 2013 IEEE 26th International Conference on Micro Electro Mechanical Systems; 2013; pp. 307-310) describes the assembly of DNA origami structures on patterned silicon using atomic force microscopy based lithography. Further, Pearson *et al.* (Pearson, A. C. et al.; NANO Letters, 11; 2011; pp. 1981-1987) describes the chemical alignment of DNA origami structures to block copolymer patterned arrays of gold nanoparticles.

Accordingly, the technical problem underlying the present invention is to provide methods for the site-specific immobilization of DNA origami structures on solid substrates.

The solution to the above technical problems is achieved by the embodiments characterized in the claims.

In particular, the present invention relates to the following items:
1. A method for the site-specific immobilization of DNA origami structures on an optically transparent solid substrate, said method comprising the steps of:
   (a) providing at least one type of DNA origami structure having single-stranded ssNA strands protruding from the DNA origami structure;
   (b) providing a pre-patterned optically transparent solid substrate that is functionalized with ssNA capture strands, wherein the ssNA capture strands are capable of binding to the ssNA strands protruding from the DNA origami structure by Watson-Crick pairing; and
   (c) incubating the optically transparent solid substrate of step (b) with the at least one type of DNA origami structure of step (a),
   wherein different areas of the optically transparent solid substrate are functionalized with different types of ssNA capture strands, and the optically transparent solid substrate is incubated with different types of DNA origami structures having different protruding ssNA strands,
   wherein said DNA origami structures are not placed on said substrate in a randomly manner, but are immobilized to selected areas or spots on said substrate, thus providing full control over the spatial location of said DNA origami structures on said substrate,
   wherein the DNA origami structures carry one or more types of objects-of-interest (OOIs), wherein the OOls are selected from the group consisting of proteins, and peptides, and
   wherein step (c) is carried out in the presence of a buffer comprising sodium dodecylsulphate (SDS) and under conditions that (i) do not compromise the structure and integrity of the DNA origami structures, the ssNA strands, and the solid substrate, and (ii) allow for Watson-Crick pairing between the protruding ssNA strands of the origami structures and the ssNA capture strands, so that immobilization of the origami structures on the solid substrate is achieved.
2. The method according to item 1, wherein the ssNA strands and capture strands are ssDNA strands and capture strands.
3. The method according to item 1 or item 2, wherein the region of Watson-Crick pairing between the ssNA/ssDNA strands protruding from the DNA origami structures and the ssNA/ssDNA capture strands is from 11 to 15 base pairs in length.
4. The method according to any one of items 1 to 3, wherein the optically transparent solid substrate is composed of glass, mica, quartz, sapphire or polymer materials.

Described herein is the immobilization of DNA origami structures on solid substrates. To this end, structural features are present on said DNA origami structures as well as on said solid substrates, wherein said structural features are capable of binding to each other. In particular embodiments, said structural features are nucleic acid structures such as single- or double-stranded nucleic acid strands or DNA origami structures. Binding of said structures can be effected by base complementarity and respective Watson-Crick binding, sticky end ligation, stacking interactions, e.g. of geometrically complementary DNA origami structures, or enzymatic ligation to achieve fixation of the DNA origami structures on the solid substrate.

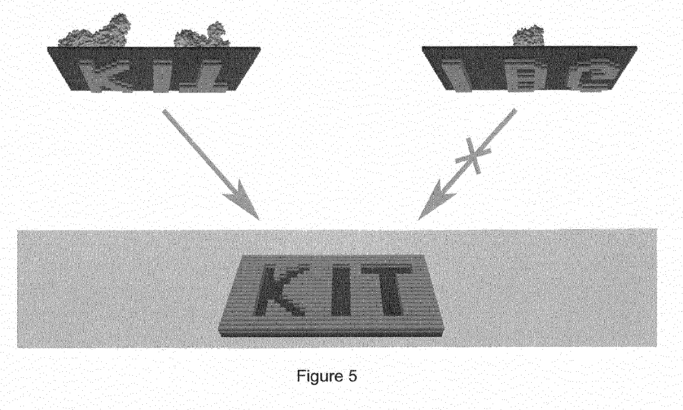

In a particular embodiment, the above first structural feature is a feature of the DNA origami structure itself having a form that is geometrically complementary to the second structural feature on the solid substrate which is a DNA origami structure that is functionalized on said solid support (Fig. 5). These geometrically complementary DNA origami structures can bind to each other by a lock-and-key mechanism that is mediated by base stacking interactions.

Methods for immobilizing DNA origami structures on a solid substrate are not particularly limited and are known in the art. They include for example top-down patterning approaches such as ink-jet printing, DPN, polymer pen lithography and the like.

According to the present invention, the above first structural feature is single-stranded nucleic acid (ssNA) strands protruding from the DNA origami structure and the above second structural feature is ssNA capture strands, wherein the ssNA capture strands are capable of binding to the ssNA strands protruding from the DNA origami structure by Watson-Crick pairing.

As used herein, the term "site-specific immobilization" refers to the fact that according to the present invention, DNA origami structures are not placed on a solid substrate in a randomly manner, but are immobilized to selected areas or spots on said substrate. This is achieved by (i) the specificity of the binding of the first and second structural features, e.g. geometrically complementary binding of DNA origami structures or Watson-Crick pairing between the ssNA strands protruding from the DNA origami structures and the ssNA capture strands on the solid substrate, and (ii) immobilizing the respective second structural features, e.g. DNA origami structures or ssNA capture strands, on selected areas and spots on said substrate. In this manner, full control over the spatial location of a given DNA origami structure on the solid substrate can be realized.

DNA origami structures (sometimes referred to herein as "origami structures" or simply "origami") for use in the method of the present invention are not particularly limited and are known in the art. Further, methods of generating DNA origami structures are well established and known in the art. Origami structures can have any two- or three-dimensional shape and typically have dimensions in the 20 to 100 nm range. However, also larger structure can be realized. In particular embodiments, the DNA origami structure can be a bundle of tubes or pipes, e.g. 3-, 6-, or 12-helix bundles, or can have a substantially flat, rectangular shape. In this context, the term "substantially" as used herein is intended to allow for certain tolerances wherein the person skilled in the art knows how these tolerances can be determined or achieved. Substantially flat, rectangular DNA origami structures typically can have surface areas of 10⁴ to 10⁶ nm². However, also smaller or larger structures can be used.

Solid substrates that can be used in connection with the present disclosure are not particularly limited and are known in the art. Further, suitable materials for the solid substrate are not particularly limited and are known in the art. According to the present invention, in the context of biological applications, the solid substrate is optically transparent. This can be achieved by choosing solid substrates that are composed of mica, glass, quartz, sapphire or suitable polymer materials. Further, in particular embodiments it is preferred that the solid substrate is flat with a surface roughness Rₐ of at most 2 nm. In this manner, the solid substrate can be used for optical microscopy as well as atomic force microscopy (AFM).

The term "comprising/comprises" as used herein is expressly intended to encompass the terms "consisting/consists of" and "consisting/consists essentially of". In particular, the terms "comprising/comprises", "consisting/consists of" and "consisting/consists essentially of" are intended to be used interchangeably herein.

The ssNA strands protruding from the DNA origami structures, as well as the ssNA capture strands on the solid substrate, are preferably ssDNA strands and ssDNA capture strands. Further, ssNA strands and ssNA capture strands can be unmodified or modified ssNA strands. Suitable modifications are not particularly limited and are known in the art. They include for example Azo-modifications known in the art which can be used to realize photoresponsive and reversible binding and unbinding of the ssNA strands protruding from the origami structures to the ssNA capture strands. Further modifications include modifications that modulate the strength of the interaction between the strands and capture strands, such as backbone modifications that alter the surface charge (e.g. peptide nucleic acids (PNA)) or structural flexibility to induce hybridization efficiency (e.g. locked nucleic acids (LNA)). Even further modifications include the use of photocleavable structures known in the art.

In specific embodiments, the ssNA strands protruding from the DNA origami structures can be hybridized with short assistant nucleic acid strands to minimize unwanted interactions with the DNA origami structure. The length of these assistant strands which also protrude from the DNA origami structures can range from one fifth to one half of the length of the respective ssNA strands. However, these assistant strands are by no means necessary for efficient DNA origami immobilization.

Preferably, the region of Watson-Crick pairing between the ssNA strands protruding from the DNA origami structures and the ssNA capture strands is from 8 to 16, more preferably from 10 to 15, more preferably 12 or 13 base pairs in length. Further, DNA origami structures can have at least 1 or at least 2, preferably from 2 to 20, more preferably from 5 to 15, more preferably from 7 to 11 protruding ssNA strands. In this context, the number of ssNA strands positively correlates with the binding strength of the DNA origami structures to the solid substrate, and also correlates with the efficiency of binding to the solid substrate. Accordingly, the number of ssNA strands can be chosen freely to accomplish a desired binding strength and/or a desired hybridization efficiency depending from the particular application of interest.

Methods for generating DNA origami structures having ssNA strands protruding from the structure are not particularly limited and are known in the art. In particular, said ssNA strands can be incorporated into the design of the structure from the very beginning. Further, ssNA strands can be appended to DNA origami structures after assembly of the latter, e.g. by using conventional linker pairs known in the art such as Streptavidin-biotin or the like.

In this context, in particular embodiments, the ssNA strands protrude from the DNA origami structure from only particular regions of the surface of the structure, so that said ssNA strands protrude into substantially the same direction. As an example, in case of flat, rectangular DNA origami structures, the ssNA strands can protrude form one face of said structure. Again, the term "substantially" as used in this context is intended to allow for certain tolerances which can be easily determined by the person skilled in the art.

The term "pre-patterned solid substrate" as used herein refers to the fact that the solid substrate is already functionalized with the second structural features, e.g. ssNA capture strands, prior to incubating the DNA origami structures with said substrate. Methods for functionalizing solid substrates with ssNA strands are not particularly limited and are known in the art. They include for example methods of directly adsorbing said ssNA strands to the substrate surface, wherein said substrate surface is preferably an activated surface, e.g. a surface activated with a hydrophobic poly-bisphenol-A-glycidyl polymer (PBAG) or hydrophilic Dendrimer intermediate layers as known in the art. Further means of activation of the substrate surface are not particularly limited and are known in the art. As the immobilization of DNA origami structures can be sensitive to the surface charge, suitable surface activation can be chosen to achieve optimal immobilization for a given application of interest.

Methods of applying the ssNA strands to the substrate surface are not particularly limited and are known in the art. They include for example ink jet spotting, dip-pen nanolithography, polymer-pen lithography, nanografting, nanocontact lithography, micro- and nanocontact printing, microfluidic deposition techniques, and electron beam lithography, wherein polymer-pen lithography is particularly preferred.

Areas on the solid substrate that are functionalized with ssDNA capture strands can have any size and shape depending on the application of interest. In particular embodiments, individual round spots having a diameter of 0.05 to 50 µm, preferably 0.1 to 10 µm, more preferably 0.1 to 2 µm, are functionalized. According to the method of the present invention, different areas of the solid substrate are functionalized with different types of second structural features, e.g. ssNA capture strands having different base sequences, and the solid substrate is incubated with different types of DNA origami structures having different first structural features, e.g. protruding ssNA strands having different base sequences. In this manner, the different types of DNA origami structures can be immobilized on different defined and selected sites, e.g. spots, on said solid substrate.

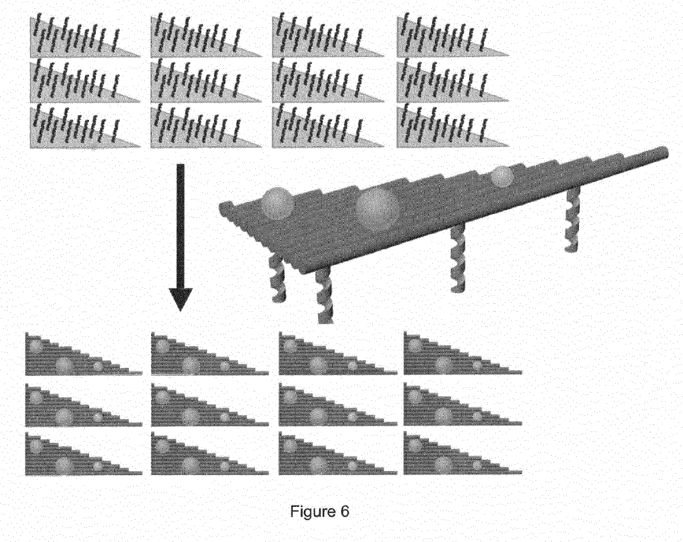

For certain applications it is preferably that a deterministic position control of the DNA origami structures on the solid substrate over millimeter length scales is achieved. This can be realized by using anisotropic shapes such as scalene triangles for the areas on the solid substrate that are functionalized. Further, DNA origami structures are used that can only fit into the anisotropic shapes in one particular orientation (Fig. 6). In this context of deterministic position control, it is preferred that the solid substrate is flat with a surface roughness Rₐ of 2 nm or less. Typical substrate materials for these applications are silicon, gold, glass, and polymer materials. Further, as the realization of anisotropic shapes requires very small capture structures, methods of applying ssNA strands to the substrate surface can include micro- and nanocontact printing or electron beam lithography. Step (c) of the method of the present invention is performed under conditions that (i) do not compromise the structure and integrity of the DNA origami structures, the first and second structural features, e.g. ssNA strands, and the solid substrate, and (ii) allow for binding of the first and second structural features, e.g. for the Watson-Crick pairing between the protruding ssNA strands of the origami structures and the ssNA capture strands, so that immobilization of the origami structures on the solid substrate is achieved. According to the present invention, step (c) of the method of the present invention is performed in the presence of a buffer containing sodium dodecylsulphate (SDS). Further, step (c) of the method can be performed at room temperature for at least 1 minute, preferably at least 1 hour, more preferably at least 2 hours, more preferably 2.5 to 3.5 hours.

According to the present invention, the DNA origami nanostructures used in the method of the present invention carry one or more types of objects-of-interest (OOIs). OOls can be located anywhere on the DNA origami structure. In particular embodiments, the OOIs are located in regions of the surface of the DNA origami structure that does not carry the first structural features, e.g. ssNA strands. Further, in particular embodiments, the OOls are presented into the direction away from the solid substrate in the final assembly. According to the present invention, said OOls are selected from the group consisting of proteins and peptides.

Methods for attaching various types of OOls to DNA origami structures are not particularly limited and are known in the art. OOls can be attached directly or indirectly. Indirect attachment can be effected by suitable binding pairs known in the art, e.g. Streptavidin-biotin, self-ligating linker proteins such as SNAP- or Halo-Tag, and antibodies, antibody mimetics or antibody fragments binding to their respective antigens. In the context of antibody fragments, single-chain antibody fragments (scFv) are particularly preferred. Each of these binding agents can be present on the DNA origami structure, with the respective binding partner present on the OOI. Means for coupling said binding agents to the DNA origami structure and the OOI are known in the art. Methods for the direct attachment of OOIs to DNA origami structures include expressed protein ligation, chemoenzymatic coupling (e.g. Sortase A coupling), coiled-coil peptide assembly, and the generation of oligonucleotide conjugates. Further, conventional homo- and heterospecific cross-linkers containing reactive groups directed against carboxyls, amines, thiols or orthogonal coupling pairs such as azide/alkyne cycloaddition or variants thereof can be used.

Further, described herein is an assembly comprising:
(i) at least one type of DNA origami structure; and
(ii) a solid substrate;
wherein the DNA origami structure has at least one type of first structural feature; the solid substrate is a pre-patterned solid substrate that is functionalized with at least one type of a second structural feature; and the second structural feature is bound to the first structural feature on the DNA origami structure.

The above structural features are present on said DNA origami structures as well as on said solid substrates, wherein said structural features are capable of binding to each other. In particular embodiments, said structural features are nucleic acid structures such as single- or double-stranded nucleic acid strands or DNA origami structures. Binding of said structures can be effected by base complementarity and respective Watson-Crick binding, sticky end ligation, stacking interactions, e.g. of geometrically complementary DNA origami structures, or enzymatic ligation to achieve fixation of the DNA origami structures on the solid substrate.

In a particular embodiment, the above first structural feature is a feature of the DNA origami structure itself having a form that is geometrically complementary to the second structural feature which is a DNA origami structure that is functionalized on the solid support. These geometrically complementary DNA origami structures are bound to each other by a lock-and-key mechanism that is mediated by base stacking interactions.

In a preferred embodiment, the above first structural feature is single-stranded nucleic (ssNA) strands protruding from the DNA origami structure and the above second structural feature is ssNA capture strands, wherein the ssNA capture strands are bound to the ssNA strands protruding from the DNA origami structure by Watson-Crick pairing. In this embodiment, the DNA origami structure has single-stranded NA (ssNA) strands protruding from the DNA origami structure; the solid substrate is a pre-patterned solid substrate that is functionalized with at least one type of ssNA capture strands; and the ssNA capture strands are bound to the ssNA strands protruding from the DNA origami structure by Watson-Crick pairing. In a particularly preferred embodiment, the ssNA strands protruding from the DNA origami structure and ssNA capture strands are ssDNA strands.

In this aspect, all relevant definitions and embodiments defined above for the method of the present invention also apply to the assembly described herein. In particular, the DNA origami structures, ssNA strands protruding from the same, solid substrates, ssNA capture strands, pairing region between said ssNA strands, and objects of interest are as defined above. Further, all preparation methods as defined above for the method of the present invention can also be employed for realizing the assembly described herein.

In a preferred embodiment, the assembly described herein is obtained by the method of the present invention.

In another preferred embodiment, the assembly described herein is part of a microfluidic device, a microarray, or a chip. Respective devices are not particularly limited and are known in the art. In particular, microfluidic systems can be developed to improve the stability of the delicate origami-modified surfaces, to ease the handling and improve the robustness of on-surface assembly and e.g. cell culture processes, and to enable multiplexing together with higher throughput capabilities. Microfluidic techniques enable simultaneous manipulation and analysis of cultured cells. Soft lithography replica molding in polydimethylsiloxane (PDMS) can be used to produce microfluidic networks, which are mounted on top and clamped or bonded to glass and polymer surface substrates, previously patterned with ssNA capture strands. NA-directed immobilization of OOI-decorated origami constructs is efficiently controllable by fluidic operations and FRET-based TIRFM and AFM analyses can be used to study these processes. Following origami immobilization, cells can be loaded fluidically with a flow of suspended cells in medium. Various designs of microreactors for origami immobilization and cell growth can be envisioned. Geometries can range from simple linear channels and channel-connected compartments to arrays of microcavities connected with fluidic liquid distributors.

Furthermore, described herein is the use of the assemblies described herein for the analysis of cells, preferably for the analysis of the cells' transmembrane proteins, more preferably for the analysis of transmembrane receptor signaling in cells, preferably in living cells.

This particular aspect has been labeled MOSAIC (Multiscale Origami Structures as Interfaces for Cells), and provides a DNA-based platform to arrange multiple different ligands with full control over their absolute number, stoichiometric proportion and orientation on the nano- and micrometer length scale and interfaces to induce well-defined architectures of signaling molecules in adhered cells. With the MOSAIC toolbox described herein, fundamental key questions in cell signaling, such as whether and how a particular size and composition of an assembly of receptors and associated molecules within the cell membrane can activate distinctive signaling pathways, can be tackled.

The importance of understanding the mechanism of early signaling goes beyond fundamental research. In particular, the full implementation of MOSAIC holds the potential to be translated into commercial products. Many disabling conditions, such as cancer and (auto)immune-related diseases, can be tracked down to the molecular function of transmembrane receptors. Thus, these proteins play an increasingly important role as druggable targets for research strategies within the pharmaceutical industry, and the understanding of spatiotemporal self-organization of receptors will help developing new strategies for pharmacological receptor targeting. There is a great need for cell-based screening assays in academic and pharmaceutical industry research. For example, MOSAIC could be used as a standardized screening platform for cell-based studies in drug development. Thus, the scope of MOSAIC in biological systems of great significance for pharmaceutical research in cancer and immune-related diseases can be explored.

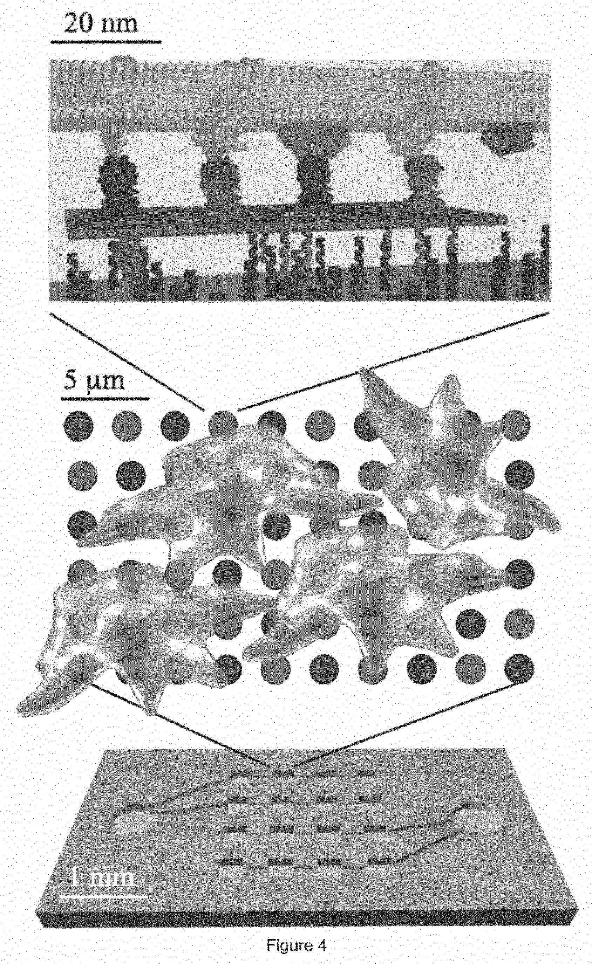

The hierarchical structure of MOSAIC (Fig. 4) is based on bottom-up assembly of scaffolded DNA origami constructs, which serve as molecular pegboards (1∼00x100 nm²) for the precise arrangement of receptor binding proteins or other ligands of signaling proteins with a lateral resolution of ± 5 nm. The origami pegboards are designed to self-assemble on DNA-encoded surface patches of -100-2000 nm feature size, produced by top-down patterning. The resulting interface contains spots (dark and light gray in Fig. 4) in which the amount, concentration, stoichiometry and nanoscale spatial relation of ligands is controlled through spot size and nature of origami (super)structures. The surface-bound ligand assemblies are capable to recruit and organize membrane proteins in adhered cells, thereby inducing distinctive homo- and heterotypic arrangements of signaling molecules which affect early cellular signaling events. The interface can be embedded in a microfluidic chip containing an array of cavities connected by smaller channels and fluidic liquid distributor systems to improve surface stability, robustness of on-surface assembly and cell culture processes, as well as multiplexing and throughput capabilities of the MOSAIC platform.

The present invention sets a sound basis for further refinement and standardization of MOSAIC as tool for fundamental research in molecular cell biology and even commercial biomedical applications. Furthermore, from the chemical and engineering point-of-view, the present invention aims at the deeper understanding of fundamental mechanisms of chemically-instructed interphase reactions between DNA nanostructures and solid surfaces. The objective is to unravel kinetic and thermodynamic parameters of these interphase reactions through systematic changes in design of DNA recognition moieties and surface composition. This basic research on DNA-directed self-assembly aims to open up novel applications for DNA nanotechnology, that is the use of DNA nanostructures as toolbox for advanced studies of biological systems.

According to the present invention, microstructuring and functionalization of a solid support surface is decoupled, such that patterning is carried out with a single class of molecules, e.g. ssNA strands, which can be immobilized under identical reagent, buffer and environmental conditions, but nonetheless have highly specific recognition properties to enable site-directed immobilization of DNA origami structures.

In contrast to top-down nanostructuring, assembly and patterning of various OOls with distances in the typical critical range of biomolecular assemblies (∼ 5 to 100 nm) can be effectively achieved in homogeneous solution through bottom-up self-assembly of DNA origami molecules. By harnessing the structure-directing properties of synthetic DNA oligonucleotides, well-defined DNA nanostructures can nowadays be assembled and used as scaffolds for the arrangement of OOIs, such as proteins, with precise control over their distance and stoichiometric composition. While several studies already underlined the feasibility and potential benefit of DNA nanoscaffolds for controlling distance and stoichiometry of ligands, this approach is still in its infancy. Moreover, soluble DNA-arranged ligand assemblies do not offer the advantages of the surface-based techniques according to the present invention, which allow for the rational tailoring of nano-to-microscale geometry of the assemblies and also permit implementation of multiplexed high-throughput tests.

The figures show:
Figure 1:
   DNA origami structures.
   a) Schematic drawing of rectangular origami plates with 60 x 120 nm² dimensions (top) and indication of selected "addressable pixels" (dark gray squares, bottom).
   b) Atomic force microscopy (AFM) images of decorated origami where pixels were addressed with proteins to generate letters. c, d) Schematic drawing of origami plates equipped with protruding ssDNA strands on one face to promote immobilization on solid substrates functionalized with complementary capture strands (light gray area in d)). The opposite face of the origami is used for the arrangement of protein binders. For simplicity of illustration, the examples show origami structures with only two different binders c) or multiple molecules of one type of binder d) arranged in a dense fashion. The sketched proteins are Fab antibody fragments, drawn to scale with the origami scaffold.
Figure 2:
   DNA-directed immobilization of DNA Origami structures.
   Schematic drawing of an origami structure containing 9 protruding ssDNA strands for immobilization and 6 Cy5 fluorophors to enable detection through fluorescence microscopy. Protruding strands for immobilization, denoted as cF1(22nt), are connected to the origami scaffold through T8 spacers. The latter are hybridized with complementary "Assistant strands" to minimize unwanted interactions with the origami scaffold. These origamis were allowed to hybridize to an array of capture oligomers of variable length, containing increasing numbers of complementary nucleotides, ranging from 2 - 19 (aF1-2 - aF1-19, respectively).
Figure 3:
   Site-specific immobilization of DNA origami structures on DNA microarravs.
   (A) A mixture of two different origami structures equipped with protruding arms to promote hybridization on either the red or green microspots was incubated on the array. Unbound material was removed and immobilized origami structures were quantified by fluorescence imaging. (B) The histogram indicates intensity of fluorescence mean values measured in individual spots for Cy3 (gray striped bars) or Cy5 (black).
Figure 4:
   Multiscale structure of DNA origami structures as interfaces for cells.
   The hierarchical structure of MOSAIC is based on DNA origami structures, which serve as molecular pegboards (-100x100 nm²) for the precise arrangement of ligands of transmembrane proteins (or other receptor binding proteins). Ligands and other OOls can be precisely arranged with a lateral resolution of ± 5 nm. The origami pegboards are designed to self-assemble on DNA-encoded surface patches of -100-2000 nm feature size, produced by top-down patterning on transparent substrates. The resulting interface will contain spots in which the amount, concentration, stoichiometry and nanoscale spatial organization of multiple ligands can be controlled through spot size and nature of the origami (super)structures. The resulting surface-bound ligand assemblies constrain in a precisely defined manner the recruitment and organization of membrane proteins in adhered cells, thereby allowing the study of early cellular signaling events. In order to allow multiplexing and high throughput capabilities, the MOSAIC will be embedded in a microfluidic chip. An array of cavities connected by fluidic channels will improve surface stability and robustness, as well as allow automated, on-surface assembly and cell culture processes.
Figure 5:
   Schematic illustration of site-specific origami immobilization through complementary shapes.
   A 3D origami with imprinted geometrical features ("KIT") is designed and assembled on the basis of 3-, 6- or 12-helix bundle structures. This origami is immobilized on solid supports by top-down patterning approaches, e.g. electron beam lithography, DPN, PPL or the like. The immobilized origami serves as capture plate for origami equipped with geometrical features of complementary shape which also carry ligands or other OOls. In this manner, stacking interactions between DNA double-helices, geometrically arranged in a shape complementary fashion, enable specific assembly of origami structures.
Figure 6:
   Deterministic position control of DNA origami structures by aid of capture arrays of anisotropic shape bearing complementary oligonucleotides.
   The origami structures are site-specifically decorated with different nanoparticles to produce long range-ordered arrays of plasmonic devices.

The present invention will be further illustrated in the following examples without being limited thereto.

### Examples

### Example 1:

### Design and assembly of DNA origami structures.

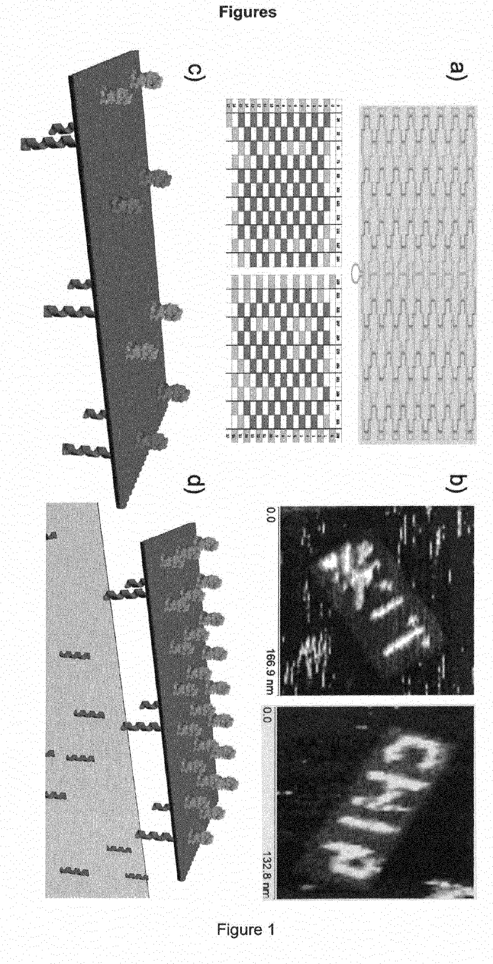

2D- and 3D-origami structures can be designed which serve as scaffold for the nanoscale arrangement of receptor-binders, such as antibodies, antibody-fragments, peptides, or small-molecules as cognate ligands for transmembrane proteins (for details, cf. Example 2). 2D rectangular origami plates represent the simplest case for such molecular pegboards (Fig. 1). The plates contain ssDNA strands protruding from one face to facilitate immobilization on surfaces functionalized with complementary capture strands. The opposite face contains functional groups which enable site-selective, orthogonal attachment of OOls such as e.g. receptor-binding ligands. In addition to quasi-2D plate-like structures, 3D origami structures based on 3-, 6- or 12-helix bundles which offer greater scaffold rigidity can be designed. Moreover, origami constructs containing structural features to enable their self-assembly into finite or infinite, periodic lattice-superstructures can be developed. Computer-aided structure design and optimized assembly reaction parameters can be used for production. Further, gel-electrophoresis, atomic force microscopy (AFM) and electron microscopy can be used for characterization of structural integrity.

### Example 2:

### Ligand decoration of DNA origami structures.

Site-selective, orthogonal methods can be used to facilitate selective arrangement and orientation of OOls such as e.g. transmembrane receptor ligands on the origami. Respective architectures can be obtained by coupling biotinylated OOls to origami-bound arrays of streptavidin (STV) molecules. If necessary, ligand density on individual STV sites can be adjusted through hybridization-based assembly of mono- or divalent STV-DNA conjugates. Protein ligands can be arrayed on origami structures using covalent methods, or preferably, genetically fused self-ligating linker proteins (e.g., SNAP- and Halo-Tag). However, the linker proteins bring with them additional steric bulk which may be disadvantageous in certain settings, for instance, in densely-packed ligand assemblies. In such cases, the direct attachment of ssDNA anchors can be realized in a site-selective manner using e.g. expressed protein ligation. The resulting ssDNA-ligand conjugates are then attached to the DNA origami scaffold through Watson-Crick hybridization. If necessary, alternative strategies for direct coupling of OOls such as proteins to origami structures can be applied, which are based on chemoenzymatic approaches (e.g., sortase A coupling) and coiled-coil peptide assembly. Further, antibodies or fragments thereof can be used as specific binders for recruitment of e.g. receptors and other signaling molecules. Although monoclonal antibodies (mABs) directed against extracellular epitopes of most transmembrane receptors are available, arrangement of whole IgG molecules on origami may not always be optimal. To avoid hardly-controllable cross-linking through homobispecific IgG and to minimize steric bulk of ligands, re-cloning of binding domains of known mABs into single-chain antibody fragments can be advantageous in selected cases.

In addition to protein ligands, other molecular components can be installed on origami constructs, e.g. to mimic cell-cell contacts by inducing the arrangement of nanostructured membrane architectures in surface-adhered cells. For example, numerous synthetic peptides and non-peptidic small-molecules are known as ligands, effectors, and inhibitors of cell signaling. Small molecule ligands can readily be incorporated into origami structures, using appropriately designed covalent oligonucleotide-conjugates. All aforementioned ligand-decorated origami constructs can be purified by established methods based on ultrafiltration, chromatography, or field-flow electrophoresis. Successful arrangement and surface occupancy of ligands on origami scaffolds can be verified by AFM analysis, and functionality of the tethered ligands can be tested by established solid-phase binding assays, e.g. immunoassays, BiaCore, and/or FACS analysis of *in vitro* cell binding.

### Example 3:

### Self-assembly of DNA origami superstructures.

To extend the design possibilities and to enlarge the area of nanoscale addressability of OOI-decorated DNA origami constructs, self-assembly approaches for the rational bottom-up fabrication of origami superstructures can be devised. Multiple DNA origami "jigsaw pieces" can be assembled into larger superstructures of finite size (∼ 300 x 350 nm²) by means of DNA hybridization. Further, jigsaw-type building blocks equipped with topographic features for directional self-assembly based on Watson-Crick hybridization and, in particular, stacking interactions can be developed. The tiles contain protruding ssDNA strands to mediate on-surface assembly and all tiles can be twist-corrected to avoid that intrinsic curvatures of individual tiles disturb assembly of superstructures on flat surfaces. In order to employ protein-decorated origami tiles, it is important that environmental parameters (*i.e*., temperature, pH, ion strength of buffers) are sufficiently mild to avoid protein denaturation. Aided by fluorescence- and FRET-based analyses, the design can be fine-tuned, such that assembly can be controlled by subtle adjustment of temperature and/or Mg²⁺ ion concentration. Assembly efficiency can be determined by electrophoresis and in situ fast scan AFM.

As alternative to finite origami superstructures methods for on-surface assembly of "infinite" periodic lattice origami superstructures can be devised through tiling approaches. To this end, typically four different origami structures represent "tiles" which can self-assemble through complementary topographical features into repetitive pseudo-2D lattices. Similar as with individual origami plates, Watson-Crick base-pairing between complementary ssDNA strands tethered to the solid surface and origami structures, respectively, are employed to facilitate immobilization and self-assembly. This approach allows (i) to fine-tune the binding strength between tiles and surface by adjustment of number, length and composition of interacting base-pairs, and (ii) to achieve site selectivity for the immobilization process, such that different sets of tiles can be directed to individual microspots bearing specific capture strands.

### Example 4:

### Immobilization and assembly of origami constructs on solid substrates.

In order to systematically explore and optimize the DNA-directed immobilization and supramolecular assembly of DNA origami constructs on surfaces, computer-aided design of capture strands and microarray-based quantification of binding efficiency by fluorescent read-out schemes is used. Fluorescence read-out is powerful not only for rapidly quantifying the impact of origami design, surface chemistry, and hybridization conditions (time, temperature, pH, ion strength) on immobilization specificity and efficiency, but also allows for monitoring self-assembly using properly positioned FRET pairs in adjacent tiles. Analysis of the ultrastructure to prove the integrity and correct assembly of various tiles can be achieved primarily by AFM. It is important to note that wide-range order of on-surface assembled nanostructures is desirable to improve control over stoichiometry and spatial relation of immobilized OOls.

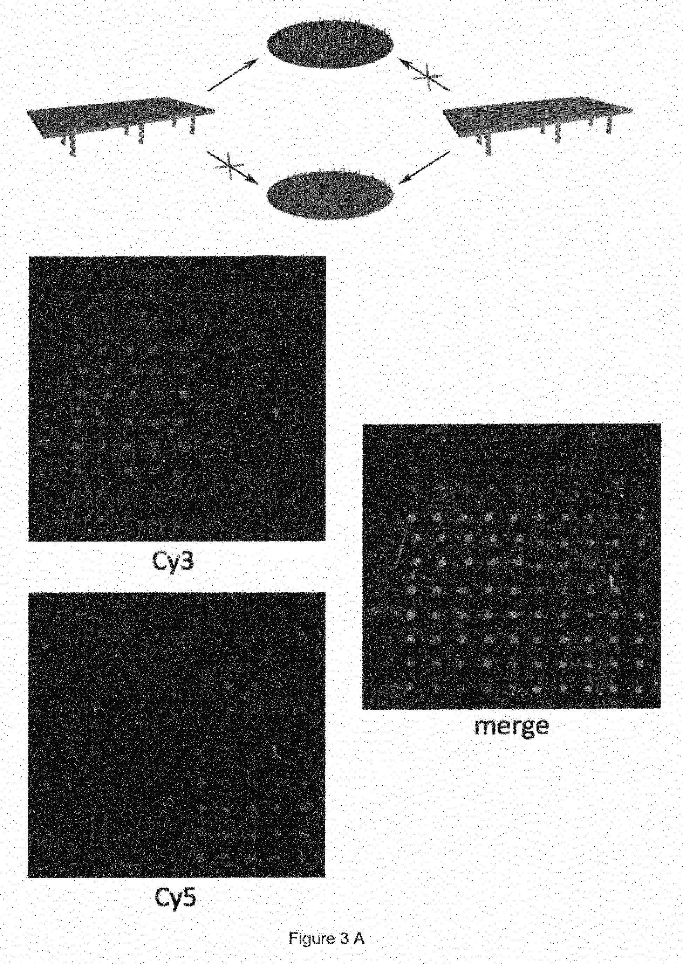

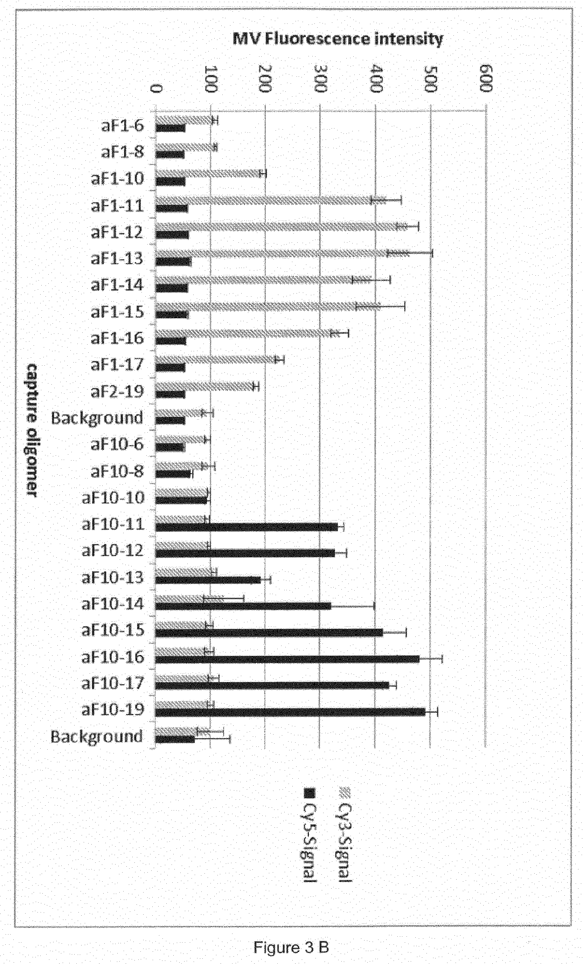

To meet the requirements of both fluorescence microscopy (transparent solid supports) and AFM (flat surfaces with roughness of ≤ 2 nm), thin mica sheets mounted on microscopy cover slides can be used. These substrates are suitable for covalent modification with ssDNA capture strands, imaging of hybridized DNA nanostructures by fluorescence microscopy and AFM, and cell culturing. Moreover, site-selective DNA-directed immobilization of origami structures has been shown (Fig. 3). On-surface assembly and immobilization can be explored by AFM analysis and characterization of on-surface assembled origami can also be achieved by (transmission) electron microscopy if required. For this purpose, individual origami tiles can be modified with DNA-coated nanoparticles.

### Example 5:

### Optimal sequence length of complementary stretches.

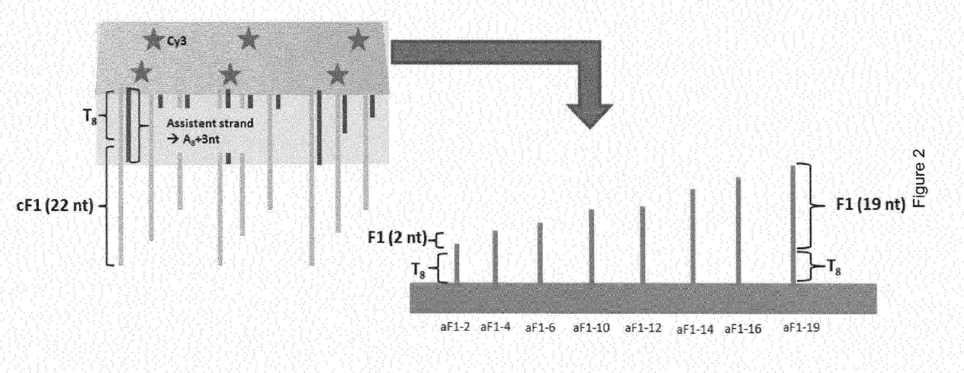

Interphase hybridization of DNA origami structures containing nine protruding ssDNA strands on one face to promote immobilization on chemically activated glass substrates bearing complementary capture strands was optimized in a microarray format. Glass surfaces were either activated with a hydrophobic PBAG or hydrophilic Dendrimer intermediate layers as known in the art. The activated glass was used to covalently bind a library of capture strands which differed in the length of the complementary region. A detailed scheme of the experiment is sketched in Figure 2.

Results indicate that the optimal sequence length of complementary stretches is in the range of 11 to 15 base pairs (Figure 3 B). Moreover, SDS (sodium dodecylsulphate) could be identified as an ingredient to suppress non-specific binding. In fact, low concentrations of SDS had a much higher impact on binding specificity than any of the usual blocking strategies (BSA, milk powder, + about 10 different conditions tested so far).

### Example 6:

### Generation of an assembly according to the present invention.

The following details an exemplary protocol for the generation of a DNA origami structure - solid substrate assembly according to the present invention.

### Origami preparation:

1) Assembly of origami in 500µl filtrated TEMg 1x with 20nM of scaffold ss109Z5 and 15 eq. of staple strands.
2) Concentrate the origami solution in Amicon Ultra 100kDa MWCO by subsequently adding 100µl of origami sample along with 400µl filtrated TEMg 1x and centrifuge 5 min at 4000g. Purify the origami solution by washing 5 times with 1ml filtrated TEMg 1x and centrifuge 5 min at 4000g. At the end concentrate the solution to about 400µl.

The concentration of the origami at the end of the procedure is expected to be 15-20nM.

### Origami-DDI:

1. Mount chamber frame around each of the capture oligomer arrays.
2. Wash the arrays with TETBS 1x to remove excess of unbound oligomers from the surface.
3. Wash a second time by incubation of the slide with TETBS 1x for 5 minutes at RT in the dark.
4. Block the slide with 50 µl of CAS-Block pro array. Incubate for 30 min on shaker at RT in the dark.
5. Wash the slide with TETBS 1x.
6. Prepare the origami solution: 2,5µl Amicon purified origami in 50µl of final volume solution. NOTE: For poly-bisphenol A carbonate (PBAC) slides add 2,5µl SDS 1% (end concentration 0,05%). For glycidyloxy-propyltrimethoxysilan (GOPTS) slides add 2,5µl CHAPS (3-[(3-cholamidopropyl) dimethylammonio]-1-propanesulfonate) 1% (end concentration 0,05%).
7. Incubate with 50 µl origami solution per array for 3hrs on shaker at RT in the dark.
8. Wash the slide with TETBS 1x.
9. Wash 2 times with 0,2x SSC-Buffer for 10 min on shaker at RT in the dark.
10. Wash the slide with TETBS 1x.
11. Wash the slide with water.
12. Dry with N2.

### Buffers:

TEMg buffer:
   20 mM Tris-CI, 2 mM EDTA, 12,5mM Mg(CH₃COO)₂ , pH 7.5
TETBS buffer:
   20 mM Tris-CI, 150 mM NaCl, 5 mM EDTA, 0,05% Tween-20, pH 7.5
20x SSC buffer:
   3 mM NaCl, 300 mM Tris,0,1% SDS, pH 7.0

## Claims

1. A method for the site-specific immobilization of DNA origami structures on an optically transparent solid substrate, said method comprising the steps of:
(a) providing at least one type of DNA origami structure having single-stranded ssNA strands protruding from the DNA origami structure;
(b) providing a pre-patterned optically transparent solid substrate that is functionalized with ssNA capture strands, wherein the ssNA capture strands are capable of binding to the ssNA strands protruding from the DNA origami structure by Watson-Crick pairing; and
(c) incubating the optically transparent solid substrate of step (b) with the at least one type of DNA origami structure of step (a),
wherein different areas of the optically transparent solid substrate are functionalized with different types of ssNA capture strands, and the optically transparent solid substrate is incubated with different types of DNA origami structures having different protruding ssNA strands,
wherein said DNA origami structures are not placed on said substrate in a randomly manner, but are immobilized to selected areas or spots on said substrate, thus providing full control over the spatial location of said DNA origami structures on said substrate,
wherein the DNA origami structures carry one or more types of objects-of-interest (OOIs), wherein the OOls are selected from the group consisting of proteins, and peptides, and
wherein step (c) is carried out in the presence of a buffer comprising sodium dodecylsulphate (SDS) and under conditions that (i) do not compromise the structure and integrity of the DNA origami structures, the ssNA strands, and the solid substrate, and (ii) allow for Watson-Crick pairing between the protruding ssNA strands of the origami structures and the ssNA capture strands, so that immobilization of the origami structures on the solid substrate is achieved.

2. The method according to claim 1, wherein the ssNA strands and capture strands are ssDNA strands and capture strands.

3. The method according to claim 1 or claim 2, wherein the region of Watson-Crick pairing between the ssNA/ssDNA strands protruding from the DNA origami structures and the ssNA/ssDNA capture strands is from 11 to 15 base pairs in length.

4. The method according to any one of claims 1 to 3, wherein the optically transparent solid substrate is composed of glass, mica, quartz, sapphire or polymer materials.

## Patentansprüche

1. Verfahren für die ortsspezifische Immobilisierung von DNA-Origami-Strukturen auf einem optisch transparenten, festen Substrat, wobei das Verfahren die Schritte umfaßt:
(a) Bereitstellen mindestens eines Typs von DNA-Origami-Struktur, welche einzelsträngige ssNA-Stränge aufweist, die von der DNA-Origami-Struktur hervorstehen,
(b) Bereitstellen eines vorab gemusterten, optisch transparenten, festen Substrats, das mit ssNA-Fangsträngen funktionalisiert ist, wobei die ssNA-Fangstränge befähigt sind, durch Watson-Crick-Paarung an die ssNA-Stränge zu binden, die von der DNA-Origami-Struktur hervorstehen und
(c) Inkubieren des optisch transparenten, festen Substrats aus Schritt (b) mit dem mindestens einen Typ von DNA-Origami-Struktur aus Schritt (a),
wobei verschiedene Bereiche des optisch transparenten, festen Substrats mit verschiedenen Typen von ssNA-Fangsträngen funktionalisiert sind und das optisch transparente, feste Substrat mit verschiedenen Typen von DNA-Origami-Strukturen inkubiert wird, welche verschiedene hervorstehende ssNA-Stränge aufweisen,
wobei die DNA-Origami-Strukturen nicht in zufälliger Weise auf dem Substrat platziert werden, sondern auf ausgewählten Bereichen oder Spots auf dem Substrat immobilisiert werden, wodurch vollständige Kontrolle über die räumliche Lokalisierung der DNA-Origami-Strukturen auf dem Substrat erreicht wird, wobei die DNA-Origami-Strukturen einen oder mehrere Typ(en) von Zielobjekt(en) (*objects-of-interest;* OOIs) tragen, wobei die OOIs ausgewählt sind aus der Gruppe, bestehend aus Proteinen und Peptiden, und
wobei Schritt (c) in Anwesenheit eines Puffers ausgeführt wird, umfassend Natriumdodecylsulfat (SDS), und unter Bedingungen, die (i) die Struktur und Integrität der DNA-Origami-Strukturen, der ssNA-Stränge und des festen Substrats nicht beeinträchtigen und (ii) Watson-Crick-Paarung zwischen den hervorstehenden ssNA-Strängen der Origami-Strukturen und den ssNA-Fangsträngen ermöglichen, sodass Immobilisierung der Origami-Strukturen auf dem festen Substrat erreicht wird.

2. Verfahren nach Anspruch 1, wobei die ssNA-Stränge und -Fangstränge ssDNA-Stränge und -Fangstränge sind.

3. Verfahren nach Anspruch 1 oder Anspruch 2, wobei der Bereich der Watson-Crick-Paarung zwischen den ssNA/ssDNA-Strängen, die von den DNA-Origami-Strukturen hervorstehen, und den ssNA/ssDNA-Fangsträngen 11 bis 15 Basenpaare Länge aufweist.

4. Verfahren nach einem der Ansprüche 1 bis 3, wobei das optisch transparente, feste Substrat aus Glas, Glimmer, Quarz, Saphir oder Polymermaterialien besteht.

## Revendications

1. Procédé d'immobilisation site spécifique de structures d'origami ADN sur un substrat solide optiquement transparent, ledit procédé comprenant les étapes de :
(a) fourniture d'au moins un type de structure d'origami ADN ayant des brins de ssNA simple brin faisant saillie de la structure d'origami ADN ;
(b) fourniture d'un substrat solide optiquement transparent pré-façonné qui est fonctionnalisé avec des brins de capture de ssNA, dans lequel les brins de capture de ssNA sont capables de se lier aux brins de ssNA faisant saillie de la structure d'origami ADN par appariement de Watson-Crick ; et
(c) incubation du substrat solide optiquement transparent de l'étape (b) avec l'au moins un type de structure d'origami ADN de l'étape (a),
dans lequel différentes zones du substrat solide optiquement transparent sont fonctionnalisées avec différents types de brins de capture d'ANss, et le substrat solide optiquement transparent est mis à incuber avec différents types de structure d'origami ADN ayant différents brins d'ANss faisant saillie,
dans lequel lesdites structures d'origami ADN ne sont pas placées sur ledit substrat de manière aléatoire, mais sont immobilisées sur des zones ou points sélectionnés sur ledit substrat, permettant ainsi une maîtrise totale de l'emplacement spatial desdites structures d'origami ADN sur ledit substrat,
dans lequel les structures d'origami ADN portent un ou plusieurs types d'objets d'intérêt (OOI), dans lequel les OOI sont sélectionnés dans le groupe constitué de protéines, et de peptides, et
dans lequel l'étape (c) est réalisée en présence d'un tampon comprenant du dodécylsulfate de sodium (SDS) et dans des conditions qui (i) ne compromettent pas la structure et l'intégrité des structures d'origami ADN, des brins de ssNA, et du substrat solide, et (ii) permettent l'appariement de Watson-Crick entre les brins de ssNA des structures d'origami et les brins de capture de ssNA, de telle sorte que l'immobilisation des structures origami sur le substrat solide est obtenue.

2. Procédé selon la revendication 1, dans lequel les brins et brins de capture d'ANss sont des brins et brins de capture de ssDNA.

3. Procédé selon la revendication 1 ou la revendication 2, dans lequel la région d'appariement de Watson-Crick entre les brins de ssNA/ssDNA faisant saillie des structures d'origami ADN et les brins de capture de ssNA/ssDNA est d'une longueur de 11 à 15 paires de base.

4. Procédé selon l'une quelconque des revendications 1 à 3, dans lequel le substrat solide optiquement transparent est composé de verre, de mica, de quartz, de saphir ou de matériaux polymères.
